# EUROPEAN PATENT SPECIFICATION

(11) **EP 4 010 705 B1**
(45) Date of publication and mention of the grant of the patent: **09.07.2025**
(21) Application number: 20750679.1
(22) Date of filing: 07.08.2020
(51) Int. Cl.: G01N 33/564, G01N 33/68

(54) **METHOD OF IDENTIFYING PEPTIDE ANTIGENS AND USES THEREOF**
VERFAHREN ZUR IDENTIFIZIERUNG VON PEPTIDANTIGENEN UND VERWENDUNGEN DAVON
PROCÉDÉ D'IDENTIFICATION D'ANTIGÈNES PEPTIDIQUES ET LEURS UTILISATIONS

(30) Priority: 09.08.2019 IT 201900014571
(43) Date of publication of application: 15.06.2022
(73) Proprietor: Humanitas Mirasole S.p.A., 20089 Rozzano (MI) (IT); Humanitas University, 20072 Pieve Emanuele (MI) (IT)
(72) Inventor: KALLIKOURDIS, Marinos, 20135 MILANO (IT); MARTINI, Elisa, 21047 SARONNO (Varese) (IT); CONDORELLI, Gianluigi, 20122 MILANO (IT)
(74) Representative: Currado, Luisa
(86) International application number: PCT/EP2020/072240
(87) International publication number: WO 2021/028338

(56) References cited:
- F. J. QUINTANA ET AL: "Functional immunomics: Microarray analysis of IgG autoantibody repertoires predicts the future response of mice to induced diabetes", PROCEEDINGS OF THE NATIONAL ACADEMY OF SCIENCES, vol. 101, no. Supplement 2, 5 October 2004 (2004-10-05), US, pages 14615 - 14621, XP055684743, ISSN: 0027-8424, DOI: 10.1073/pnas.0404848101
- M. BRITSCHGI ET AL: "Neuroprotective natural antibodies to assemblies of amyloidogenic peptides decrease with normal aging and advancing Alzheimer's disease", PROCEEDINGS OF THE NATIONAL ACADEMY OF SCIENCES, vol. 106, no. 29, 21 July 2009 (2009-07-21), US, pages 12145 - 12150, XP055684739, ISSN: 0027-8424, DOI: 10.1073/pnas.0904866106

## Description

The present invention relates to a new method for identifying peptide antigens relevant to a disease involving T cell activation
The immune system has evolved in response to the threat of invading pathogens. Its first line of defense is innate immunity and the innate immune system, including macrophages, neutrophils and monocytes, which is rapid but non-specific. Innate immune cells can eliminate pathogens, whilst they respond to and also release in a positive feedback loop, pro-inflammatory cytokines.

Adaptive immunity and the adaptive immune system (composed of T and B lymphocytes, termed T and B cells) is utilized to deal with persistent pathogens. It requires several days to become activated. T and B cells have a key feature that distinguishes them from innate immune cells. They express antigen receptors: T cell receptor (TCR) on T cells and B cell receptor (BCR) on B cells. The TCR and BCR have binding sites of variable sequence, unique in every different T cell or B cell, the result of a variability-generating somatic recombination process. As a result, each different T or B cell will have TCRs or BCRs with unique binding sequence, that bind antigens, i.e. mostly protein fragments that are present on invading pathogens. Thus, a population of T and B cells with a repertoire of variable antigen specificities will enable recognition and initiation of a response specific for any possible invading pathogen. This specificity, coupled with lymphocyte longevity, enables T and B cells to maintain long-lasting memory responses, a feature that enables vaccines to function. In the unfortunate case where the antigen does not belong to an invading pathogen, but it is expressed by the body's own cells, the adaptive response allows autoimmunity to occur (Kallikourdis M, (2018) "T cell responses to tumor: how dominant assumptions on immune activity led to a neglect of pathological functions, and how evolutionary considerations can help identify testable hypotheses for improving immunotherapy", Cancer Immunol Immunother. 67(6):989-998). In this case, the memory capacity of the T and B cells, coupled to the persistence of the self-antigen, which cannot be destroyed without destroying the organism, renders the disease chronic. These chronic processes, thus, are involved in the erroneous immune responses against the self, which occurs in autoimmunity.

Yet these processes have now also been found to contribute to many other high-morbidity and high-mortality diseases.

To classify as autoimmunity, a disease must satisfy a number of criteria, a summary of which is:
(i) the disease must be demonstrated to be transferrable if one transfers T cells from a sick individual to a healthy individual;
(ii) the disease must be demonstrated to be transferrable if one transfers (auto)antibody-containing serum from a sick individual to a healthy individual;
(iii) the disease must be demonstrated to be inducible if one immunizes a healthy individual with the self-antigens against which the autoimmune T cells and B cells are responding to.

The above-illustrated criteria are well-known (Rose, N. R. et al. (1993) "Defining criteria for autoimmune diseases", Immunology today: 426-430).

Acquired diseases, such as, for example, heart failure and Alzheimer's disease, which usually appear with aging, are not generated as a result of an erroneous autoreactive T cell or B cell response, rather arise as a result of stress within the affected tissue. Therefore, these diseases are not considered autoimmune. For instance, in the case of heart disease, stress in the heart can arise from a number of lifestyle- or environment-related risk factors, such as unhealthy diet (Type 2 Diabetes) or high blood pressure, which damage the cardiomyocytes, setting off a pathogenic process (Bui, A. L. et al. (2011), "Epidemiology and risk profile of heart failure", Nat Rev Cardiol, 8 (1), 30-41).

In the last decades, scientific work has revealed that adaptive immunity plays a role in the processes mediating the effects of many ailments in humans, from metabolic disorders, to cancer, age-related diseases and neurodegenerative/neurological disorders. Cardiovascular disease (CVD) is no exception (Ait-Oufella H. et al., (2006) "Natural regulatory T cells control the development of atherosclerosis in mice", Nat. Med., 12(2), 178-80). Adaptive immunity may have a feature that renders it especially susceptible to non-optimized function, especially in middle- to late-age diseases. Aluvihare VR et al., (2004) "Regulatory T cells mediate maternal tolerance to the fetus" Nat Immunol, 5(3), 266-71 has previously demonstrated that mammals require the anti-inflammatory arm of adaptive immunity, the regulatory T cells (Treg), in order to be able to reproduce via placental pregnancy. This is because the fetus is immunologically half-father. Without an egg to separate them, the pro-inflammatory arm of the maternal adaptive immune system would recognize the fetus as foreign and eliminate it. Treg actively and specifically suppresses this rejection. Importantly, the fully functional form of Treg only evolved in monotremes, which are egg-laying mammals, and in mammals, suggesting that reproduction may have been a key evolutionary driver for the development of the immunosuppressive arm of adaptive immunity. A corollary of this postulate is that little evolutionary pressure may be exerted on adaptive immunity after the reproductively active age of females. Thus, adaptive immunity may act non-optimally in any disease that has high incidence late in life, after the age range when reproduction usually occurs. Whilst the same argument can be applied to a lesser extent to innate immunity, the pregnancy-specific function of adaptive immunity produces a cliff-edge effect at middle age, and effects were identified in autoimmunity (Munoz-Suano A. et al, (2012) "Regulatory T cells protect from autoimmune arthritis during pregnancy", J Autoimmun, 38(2-3),J103-8), age-related inflammation (Benedusi, V, et al. (2015), "Ovariectomy shortens the life span of female mice", Oncotarget, 6(13), 10801-11), cancer (Kallikourdis M, (2018) "T cell responses to tumor: how dominant assumptions on immune activity led to a neglect of pathological functions, and how evolutionary considerations can help identify testable hypotheses for improving immunotherapy", Cancer Immunol Immunother. 67(6):989-998), and in the last four years, Heart Failure (HF) (Kallikourdis, M, et al. (2017), "T cell costimulation blockade blunts pressure overload-induced heart failure", Nat Commun, 8 14680). Most CVD ailments are associated with aging and inflammation, fitting this pattern. Thus, non-optimal function of adaptive immunity may contribute to CVD, thereby offering an efficient therapy target, especially given the role of adaptive immunity in regulating the chronic nature of immune responses, as well as to all the diseases associated with inflammation, such as e.g. metabolic diseases (diabetes not type 1, metabolic syndrome), obesity, neurodegenerative and neurological diseases (Amyotrophic Lateral Sclerosis-ALS, Alzheimer's Disease, Dementia, Age-related Dementia, Mild Cognitive Decline, Parkinson's Disease), and old-age-related diseases.

Cardiovascular diseases, neurodegenerative diseases and cancer are key causes of morbidity and mortality. Importantly, ailments from these three groups of diseases often appear in the clinic simultaneously, in a condition termed multimorbidity. Multimorbidity is an increasingly evident clinical problem, often very difficult to both diagnose and treat, as the division of healthcare into specialized disciplines renders cross-discipline diagnosis almost impossible to apply.

Multimorbidity condition may be either due to tissue degeneration leading to multi-system dysregulation, or it may be iatrogenic, i.e. caused by effects of a drug on a tissue different from the target tissue of the initial disease. Indeed, as more "biological" drugs enter clinical practice, one cannot exclude the possibility that aspects of multimorbidity can occasionally even be iatrogenic, i.e. caused by side-effects of pharmacological treatment. This is because current clinical practice has been developed around single disease-patients, often with little understanding of "off-target" effects. With biological and immunomodulating drugs, "off-target" effects may indeed be legitimate effects of the drugs' mechanism of action, albeit on off-target tissues. Hence, incomplete consideration of these effects may contribute to multimorbidity. Evidence suggests that inflammation and immune responses are implicated in the pathogenesis of all three disease groups listed above. The inherent mobility of the immune system, and its association with most chronic disease forms, renders it a potential contributing mechanism (a "common denominator") of multimorbidity.

As an example, very recent clinical observations have brought to light that cancer patients undergoing immunotherapy to unleash the activity of their T cells against a tumor, occasionally suffer from T cell- and macrophage-mediated lethal myocarditis (Heinzerling, L, et al. (2016), "Cardiotoxicity associated with CTLA4 and PD1 blocking immunotherapy", J Immunother Cancer, 4 50.; Johnson, DB, et al. (2016), "Fulminant Myocarditis with Combination Immune Checkpoint Blockade", N Engl J Med, 375(18), 1749-55). As tumor immunotherapy is increasingly applied in the clinic, this may represent an emergent, iatrogenic, immune system-dependent form of multimorbidity involving cancer and cardiac disease.

Heart Failure (HF) is the end stage of several cardiac diseases. It can occur as a result of volume or pressure overload, brought about by aortic stenosis or hypertension or other causes. Alternatively, it can occur as a result of cardiomyocyte damage, brought about by genetic mutations or by myocardial infarction (MI) injury. HF can be characterized by a reduction in left ventricle ejection fraction (HFrEF) or preserved ejection fraction (HFpEF), the two conditions being currently roughly equally prevalent among patients. The defects in cardiac functionality are observed as systolic and/or diastolic ventricular function. A small percentage (<5%) of HF cases are the result of inflammatory myocarditis, a cardiac inflammation that is the result of autoimmune responses or immune responses to viruses infecting the heart.

Clinical data generated in the last two decades show that pressure overload HF and MI-induced HF are also characterized by inflammation, as the stressed cardiomyocytes release pro-inflammatory cytokines such as TNFa, IL1b and IL6 (Shioi, T, et al. (1997), "Increased expression of interleukin-1 beta and monocyte chemotactic and activating factor/monocyte chemoattractant protein-1 in the hypertrophied and failing heart with pressure overload", Circ Res, 81(5), 664-71; Hofmann, U and S Frantz (2013), "How can we cure a heart "in flame"? A translational view on inflammation in heart failure", Basic Res Cardiol, 108(4), 356). This is in agreement with the observation that during pathological hypertrophy, a state preceding HF, a key event in the ailing heart is fibrosis, which is an immune-mediated phenomenon (Wynn, TA (2003),"IL-13 effector functions", Annu Rev Immunol, 21: 425-56). Cells of the innate immune system (macrophages) have been found to contribute to pathological cardiac inflammation (Mann, DL (2015), "Innate Immunity and the Failing Heart: The Cytokine Hypothesis Revisited", Circ Res, 116(7), 1254-68; Patel, B, et al. (2018), "CCR2+ Monocyte-Derived Infiltrating Macrophages Are Required for Adverse Cardiac Remodeling During Pressure Overload", JACC Basic Transl Sci, 3(2), 230-44).

Early attempts to therapeutically block cytokine function (TNFa) in HF failed, for various reasons, possibly including the redundancy of pro-inflammatory cytokine function. The extension of pro-inflammatory cytokine studies have more recently led to more successful clinical trials using inhibition of IL1b in a cardiac disease context (Ridker, PM, et al. (2017), "Anti-inflammatory Therapy with Canakinumab for Atherosclerotic Disease", N Engl J Med, 377(12), 1119-31). Yet this effect was still not efficient enough to warrant FDA approval.

However, as inflammation is demonstrably involved in the progression of HF, a better therapeutic target that can inhibit the deleterious inflammatory process would still have substantial potential for clinical benefit.

As outlined above, adaptive immune cells (T and B cells) act in an antigen-specific and chronic manner, meaning that they may be key regulators of long-lasting responses and thus of the diseases which involve them, such as chronic conditions, for example HF (including pressure overload-induced HF, congestive HF, age-related HF). Indeed, Nevers and co-workers (Nevers, T, et al. (2015), "Left Ventricular T-Cell Recruitment Contributes to the Pathogenesis of Heart Failure", Circ Heart Fail, 8(4), 776-87), and Laroumanie and co-workers (Laroumanie, F, et al. (2014), "CD4+ T cells promote the transition from hypertrophy to heart failure during chronic pressure overload", Circulation, 129(21), 2111-24) recently demonstrated that ablation of T cells, either in gene-deficient mice or by antibody depletion, had protective effects from HF induced by Transverse Aortic Constriction (TAC), the gold-standard mouse model for the preclinical study of HF (Rockman, HA, et al. (1991), "Segregation of atrial-specific and inducible expression of an atrial natriuretic factor transgene in an in vivo murine model of cardiac hypertrophy", Proc Natl Acad Sci USA, 88(18), 8277-81). In parallel, Kallikourdis and co-workers (Kallikourdis, M, et al. (2017), "T cell costimulation blockade blunts pressure overload-induced heart failure", Nat Commun, 8 14680) demonstrated that inhibition of T cell activation, via co-stimulation blockade with recombinant molecule CTLA4-Ig, even if administered late in the course of disease, can block progression of HF in the TAC model. The effect obtained was several-fold stronger than that achieved by standard b-blocker therapy. The cardiotoxic effect of the T cells involved the action of cardiac macrophages and affected the extent of cardiac fibrosis and cardiomyocyte viability. This was blocked by treatment with T cell co-stimulation blocker CTLA4-Ig. The presence of T cells was identified in both biopsies of human patients with HFrEF as well as HFpEF, whilst T cells are also implicated in age-related HF. These findings demonstrate that T cells are necessary for the progression of HF, even when the cause of HF is not immunological.

This cardiotoxic function of T cells may be inhibited by anti-inflammatory Treg cells (Kanellakis, P, et al. (2011), "CD4+CD25+Foxp3+ regulatory T cells suppress cardiac fibrosis in the hypertensive heart", J Hypertens, 29(9), 1820-28), as well as by the molecule CD73 (Borg, N, et al. (2017), "CD73 on T Cells Orchestrates Cardiac Wound Healing After Myocardial Infarction by Purinergic Metabolic Reprogramming", Circulation, 136(3), 297-313). T cells are also important for the progression of heart failure following myocardial infarction (Hofmann, U, et al. (2012), "Activation of CD4+ T lymphocytes improves wound healing and survival after experimental myocardial infarction in mice", Circulation, 125(13), 1652-63; Weirather, J, et al. (2014), "Foxp3+CD4+ T Cells Improve Healing after Myocardial Infarction by Modulating Monocyte/Macrophage Differentiation", Circ Res, 115:55-67).

Despite growing evidence of the cardiotoxic activity of T cells, the antigen specificity of naturally occurring T cell responses mediating non-autoimmune heart failure is still not known.

So far, studies on the antigen specificity of the responses in heart failure (HF) have mainly focused on autoimmune myocarditis, which however represents a very small percentage (<5%) of HF cases. In this pathology, a defect of the immune system results in an autoimmune response that happens to target the heart (Caforio, AL, et al. (1992), "Identification of alpha- and beta-cardiac myosin heavy chain isoforms as major autoantigens in dilated cardiomyopathy", Circulation, 85 (5), 1734-42; Caforio, AL, et al. (2015), "Passive transfer of affinity-purified anti-heart autoantibodies (AHA) from sera of patients with myocarditis induces experimental myocarditis in mice", Int J Cardiol, 179 166-77; Basavalingappa, RH, et al. (2016), "Identification of an Epitope from Adenine Nucleotide Translocator 1 That Induces Inflammation in Heart in A/J Mice", Am J Pathol, 186 (12), 3160-75; Krishnan, B, et al. (2017), "Branched chain α-ketoacid dehydrogenase kinase 111-130, a T cell epitope that induces both autoimmune myocarditis and hepatitis in A/J mice", Immun. Inflamm. Dis, 5 (4), 421-34).

US2014/0271694 discloses that the alpha isoform of myosin heavy chain raises a T cell immune response and IgG autoantibodies in autoimmune or pathogen-induced inflammatory cardiovascular diseases such as myocardial-infarction related autoimmunity (post-infarction autoimmune syndrome, PIA) and inflammatory myocarditis.

In other studies aiming at elucidating immune responses against the heart, artificial model antigens were used, rendering them not relevant to the antigens that "physiologically" drive responses in heart failure, which arise naturally during the onset of this disease (Gröschel, C, et al. (2017), "T helper cells with specificity for an antigen in cardiomyocytes promote pressure overload-induced progression from hypertrophy to heart failure", Sci Rep, 7 (1), 15998).

There is therefore a strong need of providing new effective therapeutic approaches aiming at preventing the onset of heart failure or reducing the severity of this disease.

There is also a strong need of providing a diagnostic method for diagnosing heart failure, particularly heart failure not resulting from autoimmune myocarditis.

More particularly, there is a need of providing a diagnostic method that enables to ascertain with a high degree of accuracy the presence of heart failure disease in a subject and/or the propensity of a subject to develop this disease, thereby allowing the prescription of lifestyle and/or medication choices which may further reduce the risk of developing heart failure.

These and other needs are met by the method, as defined in the appended claims, which form an integral part of the description.

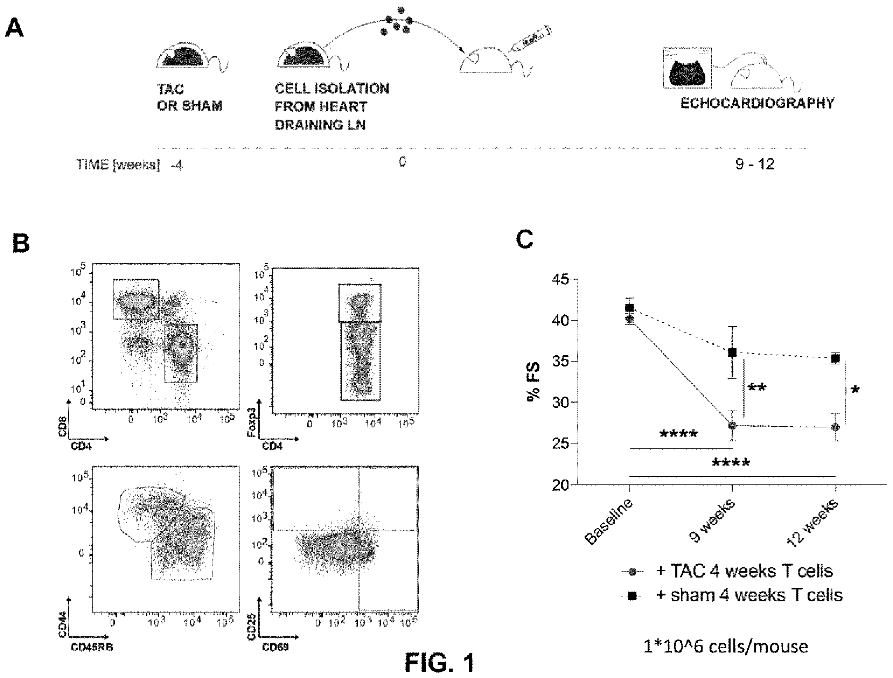

As it will be illustrated in more detail in the experimental section that follows, the present inventors have elucidated a primary role of adaptive immunity in the onset and progression of HF, even when the cause for this disease is not an immune defect. More particularly, the inventors have found out that T cell activation is not only necessary to induce heart disease, as previously shown in Kallikourdis, M, et al. (2017), "T cell costimulation blockade blunts pressure overload-induced heart failure", Nat Commun, 8 14680, but that such activation is also sufficient to induce disease progression. As shown in Figure 1, the transfer via intravenous injection of T cells taken from cardiopathic mice subjected to Transverse Aortic Constriction (TAC) to induce HF, into healthy recipient mice lead to the development of significant heart dysfunction in the latter, thus indicating the T-cell dependence of the mechanism of HF progression.

Typically, the identification of antigens recognized by T cells is a costly and technically challenging task, as the standard procedure requires the steps of identifying the T Cell Receptor (TCR) sequence of the T cells involved in the disease, cloning and expressing the sequence of each of the two independent TCR chains so that they fold together, and scanning the generated TCRs against peptide libraries. Moreover, as the TCR recognizes only peptides processed for and expressed on Major Histocompatibility (MHC) molecules, which are highly polymorphic and change from person to person, the TCR-peptide screen may produce results that are limited in their validity to small genetically similar populations, thus with limited applicability.

In order to overcome the drawbacks and limitation of the methods of the prior art, the present invention now provides a new method of identifying peptide antigens that drive the T cell responses in adaptive immunity involved in the onset and progression of a non-autoimmune disease, particularly in the chronicity of a non-autoimmune disease.

The method of the invention is based on the surprising finding made by the inventors that the antigen specificity of IgG-switched antibodies may be used as a proxy for the antigen specificity of T cells that were required to enable B cells to produce these antibodies, in the context of a T cell-dependent non-autoimmune disease.

Antibodies against a specific antigen, produced by B cells that have interacted with said antigen, switch their heavy chain class (from IgM or IgD, to IgG or IgA or IgE) only if the B cells interact, i.e. receive "help", with a T cell recognizing the same specific antigen. Both T cells and B cells have variable antigen receptors, each unique in every T or B cell, capable of recognizing any antigen. Upon interacting with a T cell recognizing the same antigen, antibody-producing B cells can switch their antibody heavy chains from IgM or IgD to IgG, or IgA or IgE. Hence, IgG-switched antibodies guarantee that the antigen-specificity of the producing B cell matches that of an activated T cell.

The invention is a method for identifying a peptide antigen which is relevant to a human or veterinary non-autoimmune disease involving T cell activation, said method comprising the following steps:
(i) contacting a first biological fluid sample containing IgG immunoglobulins from a first non-human animal affected by the non-autoimmune disease with a microarray comprising a plurality of isolated or synthesized peptide antigens, each peptide having a pre-determined location in the microarray and comprising an amino acid sequence of a protein from the non-human animal;
(ii) detecting the binding of one or more of the IgG immunoglobulins present in the first sample with one or more of the peptide antigens in the microarray to provide a first IgG-bound peptide profile;
(iii) comparing the first IgG-bound peptide profile to a second IgG-bound peptide profile generated by contacting a microarray as defined in step (i) with a second biological fluid sample containing IgG immunoglobulins from a second non-human animal, wherein the second non-human animal is not affected by the non-autoimmune disease and wherein the first and second non-human animals belong to the same species and are congenic animals;
(iv) identifying the one or more IgG-bound peptides which are present in the first IgG-binding profile and are not present in the second IgG binding profile;
(v) querying a database of animal protein sequences using the amino acid sequence of each IgG-bound peptide identified in step (iv) as the query in order to select one or more animal proteins comprising a peptide sequence which has an amino acid sequence identity to the amino acid sequence of each of the queried peptides comprised between 67% and 100%, wherein the animal protein sequences are from an animal belonging to a species which is different from the first and second non-human animals of step (iii);
(vi) identifying as peptide antigens relevant to the non-autoimmune disease the peptide sequences which have from 67% to 100% amino acid sequence identity selected in step (v) and which are comprised in an animal protein expressed in a tissue affected by the non-autoimmune disease, wherein the non-autoimmune disease is selected from the group consisting of heart failure (HF), cardiac diseases, vascular diseases, atherosclerosis, vascular stenosis, non-type 1 diabetes, metabolic syndrome, obesity, Amyotrophic Lateral Sclerosis-ALS, Alzheimer's Disease, Dementia, Age-related Dementia, Mild Cognitive Decline, Parkinson's Disease, and any combination thereof.

According to the invention, the peptide antigen identified with the method of the invention, hereinafter designated a discovery method, is relevant to a non-autoimmune disease involving T cell activation, which means that the peptide antigen is sufficient to induce said non-autoimmune disease or may be involved in the progression of said non-autoimmune disease in a human being or in an animal.

The discovery method of the invention makes use of a microarray comprising a plurality of isolated or synthesized antigenic peptides comprising an amino acid sequence of a protein from a non-human animal, for example of a murine protein.

By way of example, the amino acid sequences of said plurality of antigenic peptides may be from tissue-specific proteins of a non-human animal, such as e.g. mouse cardiac tissue-specific proteins or mouse nervous tissue-specific proteins, or from the proteome set of proteins of a non-human animal, such as e.g. the mouse proteome.

In one embodiment, the microarray employed in the discovery method of the invention may be a planar microarray, wherein the isolated or synthesized antigenic peptides are immobilized onto a solid support, at discrete individual locations. Alternatively, the microarray may be a bead-based microarray, wherein the isolated or synthesized antigenic peptides are bound to color-coded or size-coded microspheres.

According to the discovery method of the invention, the peptide microarray is assayed with a first and a second biological fluid sample containing IgG immunoglobulins, said first and second biological fluid sample being from a first non-human animal affected by a non-autoimmune disease and from a second non-human animal not affected by said non-autoimmune disease, respectively. Following the microarray assay, differentially IgG-bound antigenic peptides are identified.

A key feature of the discovery method of the invention is that the first and second non-human animals belong to the same species and are congenic, i.e. genetically identical. Such a key feature provides for any difference in the IgG antibody repertoire of these animals, and in the corresponding profile of IgG-bound peptides in the microarray, being ascribed to the non-autoimmune disease which affects the first non-human animal and does not affect the second non-human animal.

The first and second non-human animals are mice.

IThe first non-human animal is a mouse model of the non-autoimmune disease, preferably a mouse model of a cardiovascular disease, more preferably a Transverse Aortic Constriction (TAC) model.

As known in the art, TAC in the mouse is a commonly used experimental model for pressure overload-induced cardiac hypertrophy and heart failure.

Other non-limiting examples of mouse models of disease are chronic heart failure after myocardial infarction or genetically-induced models of dilated cardiomyopathy, age-related Heart Failure models (aged mice), Myocardial Infarction models, HFpEF mouse models, hypertension models leading to HF (induced by AngII or L-NAME administration).

In the discovery method of the invention, the amino acid sequence of each IgG-bound peptide identified as above described is used as a query sequence and compared with a database of animal protein sequences, said animal being of a different species than the first and second non-human animals.

The discovery method of the invention is further characterized in that an animal protein database is queried with the amino acid sequence of each IgG-bound peptide identified as above described. The results of the database query are selected according as to whether the identified animal protein comprises a peptide sequence which has an amino acid sequence identity to the amino acid sequence of each of the queried peptides comprised between 67% and 100%.

Preferably, the amino acid sequence identity is of at least 67%, at least 70%, at least 75%, at least 80%, at least 85%, at least 90%, at least 95%, at least 96%, at least 97, at least 98%, or at least 99%.

As used herein, the term "percent (%) amino acid sequence identity" with respect to a reference polypeptide sequence refers to the percentage of amino acids in a candidate sequence that are identical to the amino acids in the reference polypeptide sequence, after aligning the sequences and introducing gaps, if necessary, to achieve the maximum percent sequence identity. Alignment for purposes of determining percent amino acid sequence identity can be achieved in various ways that are within the capabilities of one of skill in the art, for example, using publicly available computer software such as BLAST, BLAST-2, or Megalign software. Those skilled in the art can determine appropriate parameters for aligning sequences, including any algorithms needed to achieve maximal alignment over the full length of the sequences being compared. For example, percent sequence identity values may be generated using the sequence comparison computer program BLAST.

As a further step of the discovery method of the invention, peptide antigens relevant to the non-autoimmune disease are identified as having an amino acid sequence comprised in an animal protein expressed in a tissue affected by the non-autoimmune disease.

In one embodiment, the animal is a human being, a dog or a cat.

Any type of biological fluid sample suitable for assaying IgG immunoglobulin content can be used for carrying out the discovery method of the invention, preferably blood samples and derivatives thereof, such as serum and plasma.

In a preferred embodiment, the detection of the binding of one or more of the IgG immunoglobulins present in the first and/or second biological fluid sample with one or more of the antigenic peptides in the microarray is achieved by means of a secondary antibody. Typically, the secondary antibody is directed towards the IgG immunoglobulin that is part of the immunocomplex formed between said IgG and the peptide, and is generated in an animal species that is different from the first and second non-human animals from whom the first and second biological fluid samples were taken.

Preferably, the secondary antibody used in the discovery method of the invention is an anti-IgG antibody, more preferably is an anti-IgG antibody labeled with a detectable label preferably selected from a radioactive isotope, a fluorophore, an enzyme, a chemiluminescent compound, an affinity label such as for example avidin or biotin.

Although the present experimental studies have been carried out using a mouse model of heart failure, it should be understood that the discovery method of the invention is directly applicable to the identification of peptide antigens relevant to other non-autoimmune diseases, particularly chronic non-autoimmune diseases likely to be T cell mediated.

**In** a preferred embodiment, heart failure is hemodynamically induced heart failure i.e. caused by hemodynamic stress on the cardiomyocytes, which includes increased workload due to post-myocardial infarction, increased pre-load or afterload due to valvular disease or arterial pressure increase or intrinsic, therefore inherited, defects of the cardiomyocyte.

**In** a still further embodiment, the discovery method of the invention may be employed as a tool for the management of multimorbidity.

As above described, multimorbidity is a clinical situation wherein a patient suffers simultaneously from more than one of the following groups of ailments: cardiovascular, neurological, oncological and metabolic disease.

According to the invention, peptide antigens may be identified which are relevant to a large range of non-autoimmune diseases involving T cell activation, such as e.g. cardiovascular, neurological, oncological and metabolic diseases, and such antigens may be used to provide a real-time assessment of propensity/early diagnosis of emerging adaptive immune responses targeting different tissues.

Thanks to the method of the invention a first sub-clinical sign of morbidity affecting these tissues may be provided, thereby enabling cross-discipline diagnosis and rapid referral to specialist with a focus on the newly-affected tissue, pre-empting the progression of multimorbidity.

Advantageously, the discovery method of the invention may be repeatedly applied until no more novel peptides are identified, thereby reaching saturation. Indeed, in most immune responses where the target is not rapidly mutating (i.e. not a virus or a tumor) only a small number of immunodominant antigens drive the response. Thus, a few runs of the discovery method of the invention will enable saturation in the identification of novel hits, for example, the identification of the majority of the antigens driving the HF-promoting adaptive response.

Thanks to the unique and advantageous features of the discovery method of the invention as defined above, the present inventors have identified novel peptides which act as drivers of adaptive immune responses to cardiac stress, particularly of the T cell responses that mediate heart failure (HF).

As is further explained in detail below, the inventors have assayed serum samples collected from congenic TAC-operated (hereinafter referred to as TAC) and sham-operated (hereinafter referred to as sham) control mice on a mouse microarray comprising known random oligopeptides. The profiles of IgG-bound peptides generated by microarray analysis were computationally filtered in a differential analysis for IgG-bound peptides present in the TAC-assayed and not in the sham-assayed microarray. The results of this analysis were subjected to a further filtering step so as to select only peptide antigens having high homology to human proteins expressed in the cardiovascular tissue.

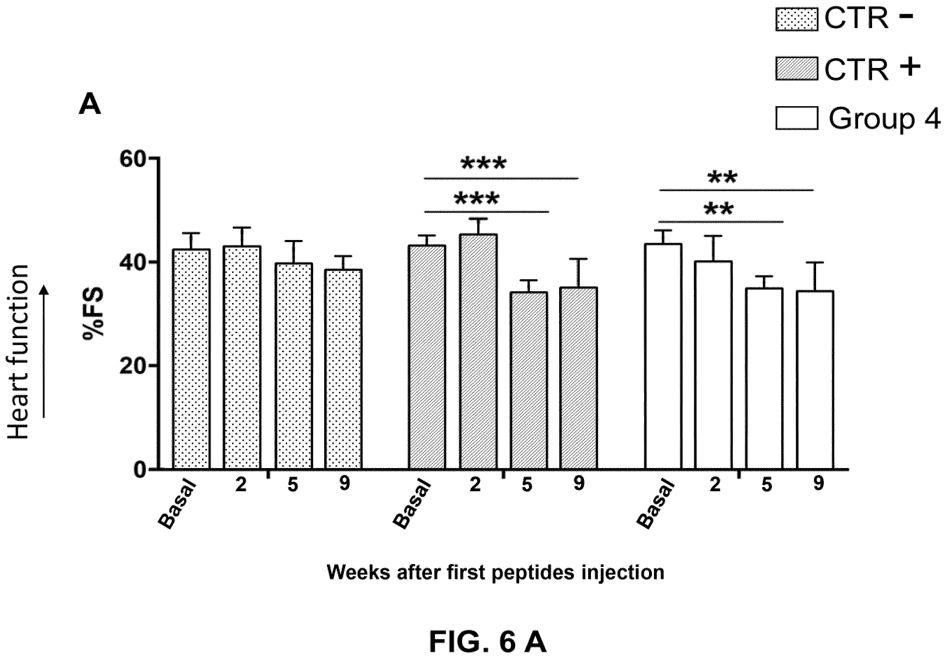

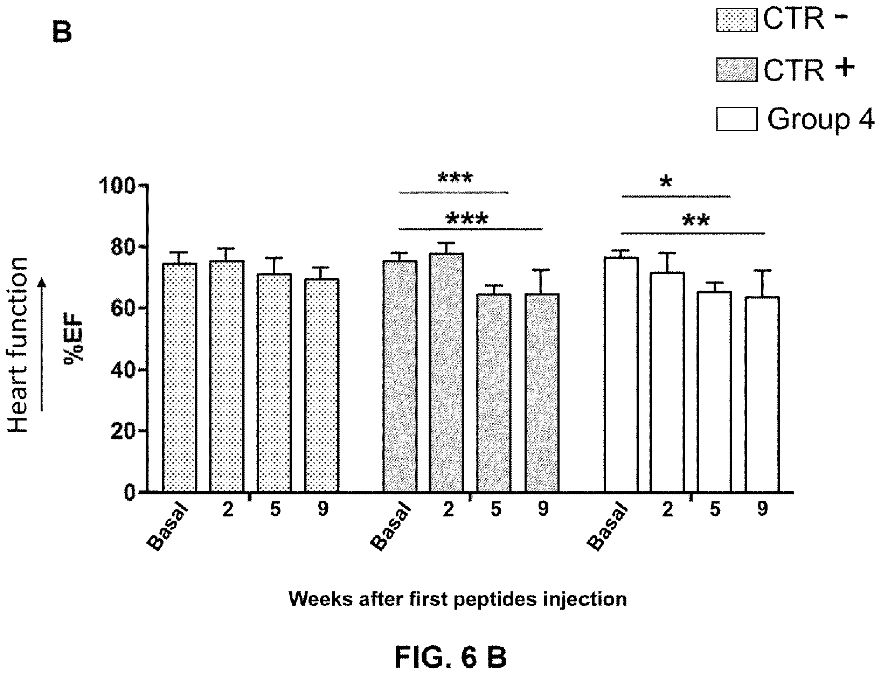

To validate the results of the above-described approach, the inventors have conducted a functional study by immunizing healthy mice with the selected peptide antigens in the presence of adjuvant, without applying any cardiovascular stress. As is shown in Figures 6A and 6B, mouse immunization surprisingly led to reduced heart function as assessed by measuring the fractional shortening (FS) and the ejection fraction (EF) in the treated animals.

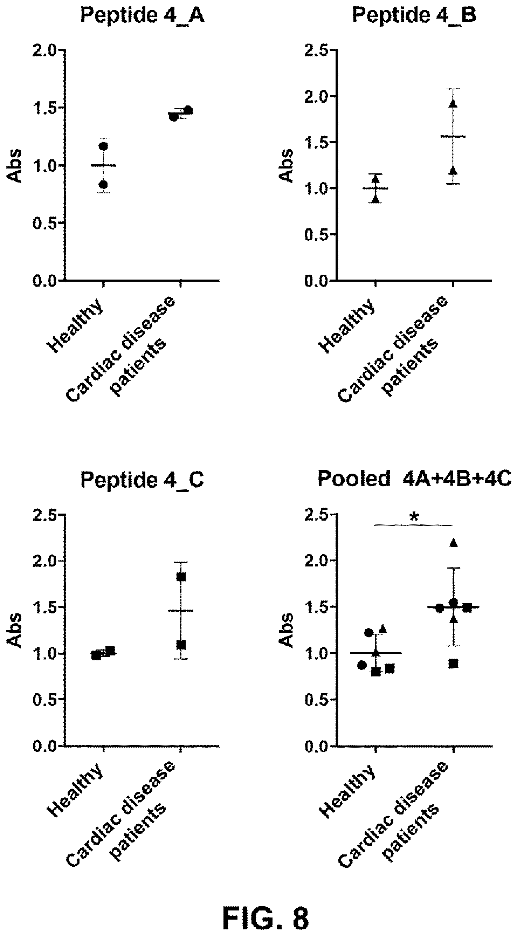

Hence, the studies conducted by the present inventors revealed for the first time that the identified novel peptides are *per se* sufficient to induce heart failure in an animal and are active mediators of disease progression, thereby representing ideal diagnostic and therapeutic markers. Further supporting the previously described experimental results, the present inventors checked the diagnostic value of the novel peptides in an ELISA immunoassay, by assaying the presence of IgG antibodies recognizing these peptides in human sera from patients affected by heart failure (HF) and healthy controls. The results from the ELISA test, illustrated by the scatter plots in Figure 8, demonstrate that measuring the titer of IgG antibodies against the novel peptides, produced by T cell-dependent B cells, enables to differentiate HF patients from healthy controls.

An isolated peptide consisting of an amino acid sequence selected from the group consisting of:
SEQ ID NOs. 1, 2, 3 and 4 of a human 14-3-3 protein; SEQ ID NO. 5 of human small nuclear ribonucleoprotein Sm D1 (SNRPD1); SEQ ID NO. 6 of human ATP synthase subunit O, mitochondrial precursor (ATP5O); SEQ ID NO. 7 of human toll-like receptor 5 precursor (TLR5), and any combination thereof;
or an isolated peptide consisting of an amino acid sequence selected from the group consisting of:
   SEQ ID NOs. 1, 2, 3 and 4 of canine 14-3-3 protein; SEQ ID NO. 8 of canine monocarboxylate transporter 3 and 4 proteins (MCT3 and MCT4); SEQ ID NO. 5 of canine SNRPD1; SEQ ID NO. 9 of canine ATP5O; SEQ ID NO. 10 of canine dihydropyrimidinase (DHP) protein, and any combination thereof;
   or an isolated peptide consisting of an amino acid sequence selected from the group consisting of:
      SEQ ID NOs. 1, 2, 3 and 4 of feline 14-3-3 protein; SEQ ID NO. 8 of feline MCT3 and MCT4 proteins; SEQ ID NO. 11 of feline vomeronasal 1 receptor felCatV1R6 protein; SEQ ID NO. 12 of feline transmembrane emp24 domain-containing protein 6; SEQ ID NO. 5 of feline SNRPD1; SEQ ID NO. 13 of feline ATP5O; SEQ ID NO. 10 of feline DHP protein, and any combination thereof.

The isolated peptide is cardiac-specific in that it consists of an amino acid sequence of a protein expressed in human, canine or feline cardiac tissue, though not excluding its possible expression also in other tissues.

The term "14-3-3 protein" as used in the present description refers to the different isoforms of human, dog and cat 14-3-3 protein including human isoforms: 14-3-3 protein epsilon, 14-3-3 protein epsilon isoform X1, 14-3-3 protein epsilon isoform X2, 14-3-3 protein beta/alpha, 14-3-3 protein theta, 14-3-3 protein zeta/delta, 14-3-3 protein eta, 14-3-3 protein gamma, 14-3-3 protein sigma; dog isoforms: 14-3-3 protein epsilon isoform X1, 14-3-3 protein epsilon isoform X2, 14-3-3 protein epsilon isoform X3, 14-3-3 protein epsilon isoform X4, 14-3-3 protein theta-like, 14-3-3 protein theta, 14-3-3 protein zeta/delta, 14-3-3 protein beta/alpha, 14-3-3 protein eta, 14-3-3 protein gamma, 14-3-3 protein sigma; and cat isoforms: 14-3-3 protein epsilon isoform X1, 14-3-3 protein epsilon isoform X2, 14-3-3 protein epsilon isoform X3, 14-3-3 protein theta, 14-3-3 protein zeta/delta, 14-3-3 protein beta/alpha, 14-3-3 protein eta, 14-3-3 protein gamma, 14-3-3 protein sigma.

The term "monocarboxylate transporter 3 (MCT3) or 4 (MCT4) proteins" as used in the present description refers to the different isoforms of human, dog and cat MCT3 and MCT4 proteins, respectively, including feline MCT4 isoform X2 and isoform X21.

The term "transmembrane emp24 domain-containing protein 6" as used in the present description refers to the different isoforms of human, dog and cat transmembrane emp24 domain-containing protein 6, including feline transmembrane emp24 domain-containing protein 6 isoform X1 and isoform X2.

The term "small nuclear ribonucleoprotein Sm D1 (SNRPD1)" as used in the present description refers to the different isoforms of human, dog and cat SNRPD1, including feline SNRPD1 isoform X1 and isoform X2.

The term "dihydropyrimidinase protein" as used in the present description refers to the different isoforms of human, dog and cat dihydropyrimidinase protein, including feline dihydropyrimidinase isoform X1 and isoform X2.

Disclosed is an in vitro method for the diagnosis and/or for predicting the risk for developing heart failure (HF) in a human subject, said method comprising the steps of:
(i) contacting a biological fluid sample containing IgG immunoglobulins derived from a human subject affected by HF or suspected of being at risk for developing HF, with one or more isolated antigenic peptides from one or more human proteins selected from the group consisting of 14-3-3 protein epsilon (SEQ ID NO. 18), 14-3-3 protein epsilon isoform X1 (SEQ ID NO. 19), 14-3-3 protein epsilon isoform X2 (SEQ ID NO. 20), 14-3-3 protein beta/alpha (SEQ ID NO. 21), 14-3-3 protein theta (SEQ ID NO. 22), 14-3-3 protein zeta/delta (SEQ ID NO. 23), 14-3-3 protein eta (SEQ ID NO. 24), 14-3-3 protein gamma (SEQ ID NO. 25), 14-3-3 protein sigma (SEQ ID NO. 26), small nuclear ribonucleoprotein Sm D1 isoform 1 (SEQ ID NO. 27), ATP synthase subunit O, mitochondrial precursor (SEQ ID NO. 28), toll-like receptor 5 precursor (SEQ ID NO. 29), and any combination thereof, wherein the one or more isolated antigenic peptides are from 6 to 50 aminoacids in length;
(ii) detecting the formation of one or more immunocomplexes between the IgG immunoglobulins in the biological fluid sample and the one or more isolated antigenic peptides, the formation of the one or more immunocomplexes being indicative of the human subject being affected by HF or being at risk for developing HF.

The one or more isolated antigenic peptides consist of an amino acid sequence selected from the group consisting of SEQ ID NOs. 1, 2, 3 and 4 of a human 14-3-3 protein; SEQ ID NO. 5 of human small nuclear ribonucleoprotein Sm D1 (SNRPD1); SEQ ID NO. 6 of human ATP synthase subunit O, mitochondrial precursor (ATP5O); SEQ ID NO. 7 of human toll-like receptor 5 precursor (TLR5), and any combination thereof.

In cases where the immune response against the diseased cardiovascular tissue exists without having yet led to clinically-observable symptoms, the detection of IgG antibodies in the in vitro method of the invention via the identified novel antigens provides a valuable indication of the propensity of the subject under examination to develop clinically-identifiable disease symptoms. If the heart disease has already developed clinically-identifiable symptoms, the detection in the method of the invention of IgG antibodies against the identified novel antigens enables the diagnosis of the specific heart disease.

The present invention is not limited to human diagnosis and finds veterinary applications as well.

Accordingly, in another non-claimed aspect, the method is an in vitro method for the diagnosis and/or prognosis of heart failure (HF) and/or for predicting the risk for developing HF in a dog, wherein the isolated antigenic peptides are from one or more canine proteins selected from the group consisting of 14-3-3 protein epsilon isoform X1 (SEQ ID NO. 30), 14-3-3 protein epsilon isoform X2 (SEQ ID NO. 31), 14-3-3 protein epsilon isoform X3 (SEQ ID NO. 32), 14-3-3 protein epsilon isoform X4 (SEQ ID NO. 33), 14-3-3 protein theta-like (SEQ ID NO. 34), 14-3-3 protein theta (SEQ ID NO. 35), 14-3-3 protein zeta/delta (SEQ ID NO. 36), 14-3-3 protein beta/alpha (SEQ ID NO. 37), 14-3-3 protein eta (SEQ ID NO. 38), 14-3-3 protein gamma (SEQ ID NO. 39), 14-3-3 protein sigma (SEQ ID NO. 40), monocarboxylate transporter 3 and 4 proteins (SEQ ID NOs. 41 and 42), small nuclear ribonucleoprotein Sm D1 (SEQ ID NO. 43), ATP synthase subunit O, mitochondrial precursor (SEQ ID NO. 44), dihydropyrimidinase protein (SEQ ID NO. 45), and any combination thereof, wherein the one or more isolated antigenic peptides are from 6 to 50 aminoacids in length.

The one or more isolated antigenic peptides consist of an amino acid sequence selected from the group consisting of SEQ ID NOs. 1, 2, 3 and 4 of canine 14-3-3 protein; SEQ ID NO. 8 of canine monocarboxylate transporter 3 and 4 proteins (MCT3 and MCT4); SEQ ID NO. 5 of canine SNRPD1; SEQ ID NO. 9 of canine ATP5O; SEQ ID NO. 10 of canine dihydropyrimidinase (DHP) protein, and any combination thereof.

This aspect may be relevant to Heart Failure from Dilated Cardiomyopathy arising due to genetic mutations in dog strains including but not limited to Doberman Pinscher, Great Dane, Boxer, Cocker Spaniel.

In a another non-claimed aspect, the method is an in vitro method for the diagnosis and/or prognosis of heart failure (HF) and/or for predicting the risk for developing HF in a cat, wherein the isolated antigenic peptides are from one or more feline proteins selected from the group consisting of 14-3-3 protein epsilon isoform X1 (SEQ ID NO. 46), 14-3-3 protein epsilon isoform X2 (SEQ ID NO. 47), 14-3-3 protein epsilon isoform X3 (SEQ ID NO. 48), 14-3-3 protein theta (SEQ ID NO. 49), 14-3-3 protein zeta/delta (SEQ ID NO. 50), 14-3-3 protein beta/alpha (SEQ ID NO. 51), 14-3-3 protein eta (SEQ ID NO. 52), 14-3-3 protein gamma (SEQ ID NO. 53), 14-3-3 protein sigma (SEQ ID NO. 54), monocarboxylate transporter 3 protein (SEQ ID NO. 55), monocarboxylate transporter 4 proteins isoform X2 (SEQ ID NO. 56) and isoform X21 (SEQ ID NO. 57), vomeronasal 1 receptor felCatV1R6 protein (SEQ ID NO. 58), transmembrane emp24 domain-containing protein 6, isoform X1 (SEQ ID NO. 59), transmembrane emp24 domain-containing protein 6, isoform X2 (SEQ ID NO. 60), small nuclear ribonucleoprotein Sm D1, isoform X1 (SEQ ID NO. 61), small nuclear ribonucleoprotein Sm D1, isoform X2 (SEQ ID NO. 62), ATP synthase subunit O, mitochondrial precursor (SEQ ID NO. 63), dihydropyrimidinase protein, isoform X1 (SEQ ID NO. 64), dihydropyrimidinase protein, isoform X2 (SEQ ID NO. 65) and any combination thereof, wherein the one or more isolated antigenic peptides are from 6 to 50 aminoacids in length.

The one or more isolated antigenic peptides consist of an amino acid sequence selected from the group consisting of SEQ ID NOs. 1, 2, 3 and 4 of feline 14-3-3 protein; SEQ ID NO. 8 of feline MCT3 and MCT4 proteins; SEQ ID NO. 11 of feline vomeronasal 1 receptor felCatV1R6 protein; SEQ ID NO. 12 of feline transmembrane emp24 domain-containing protein 6; SEQ ID NO. 5 of feline SNRPD1; SEQ ID NO. 13 of feline ATP5O; SEQ ID NO. 10 of feline DHP protein, and any combination thereof.

This aspect may be relevant to Heart Failure from Dilated Cardiomyopathy arising due to genetic mutations in cat strains including but not limited to Burmese, Abyssinian, and Siamese.

This aspect may be relevant to Heart Failure from Hypertrophic Cardiomyopathy arising due to genetic mutations in cat strains including but not limited to Maine Coon, Ragdoll, British Shorthair, Sphynx, Chartreux and Persian.

The one or more isolated antigenic peptides are from 6 to 50 amino acids in length, preferably from 7, 8, 9, 10, 15, 20, 25, 30, 35, 40, 45 to 50 amino acids in length.

Among the most largely used methods for identifying and quantifying an antibody specific against an antigen in a biological sample, the immunological procedures are the method of choice. Hence, the in vitro method o is an immunoassay.

The in vitro method is an Enzyme-Linked Immunosorbent Assay, known as ELISA. In this assay an immunocomplex is formed between the one or more antigenic peptides immobilized on a solid phase, for example the surface of a microtitre plate or a bead, and the IgG antibodies present in a biological fluid sample from the patient, such as for example a serum or a plasma sample.

The formation of IgG antibody/antigenic peptide complexes may then be detected using any suitable method. A labelled secondary anti-IgG immunoglobulin antibody is used. Suitable labels include for example fluorescent compounds, chemiluminescent compounds, radioactive compounds, enzymes and enzyme substrates, and molecules suitable for colorimetric detection.

Obviously, the use of any type of immunoassay format, the selection of which falls within the skills of the ordinary person of skill in the art.

Preferably, the biological fluid sample is selected from the group consisting of whole blood, serum, plasma, synovial fluid, follicular fluid and intraocular fluid. The biological fluid sample may optionally include further components, such as for example: diluents, preservatives, stabilizing agents and/or buffers. If needed, dilutions of the biological fluid sample are prepared using any suitable diluent buffer known in the art.

A quantitative assessment may be performed of the levels of the cardiovascular antigen-specific IgG antibodies in the patient against standards of "true" healthy controls as well as healthy controls with matching age and gender. The readout of such assessment provides an indication of the propensity/risk in a subject for developing cardiovascular disease with clinically-identifiable symptoms. In cases where an aged "healthy" individual has a non-zero cardiac-specific antibody titer, the non-zero signal, as quantified in the method of the invention against "true negatives" represents nonetheless a proxy of propensity to cardiotoxic immune responses and thus cardiovascular disease.

The in vitro method is suitable for the real-time assessment of cardiotoxicity in tumor immunotherapy.

Checkpoint blockade immunotherapy (CBI) (e.g. via antiPD1, antiCTLA4) is a novel, highly successful means of therapy of advanced hematologic and solid tumors, that is being increasingly applied in the clinic (Barbee, MS, et al. (2015), "Current status and future directions of the immune checkpoint inhibitors ipilimumab, pembrolizumab, and nivolumab in oncology", Ann Pharmacother, 49 (8), 907-37). Cardiotoxic responses are mediated by the T cell-driven mechanism. Thus, the in vitro method may advantageously enable the prediction and/or detection and quantification of T-cell responses that target cardiovascular tissues when the patient has still sub-clinical cardiac symptoms. Whilst the cardiotoxic responses affects a small, though increasing, percentage of patients, they become rapidly lethal once they appear, hence their early detection is life-saving.

The in vitro method provides for the diagnosis of potential risks associated with the introduction of novel biological drugs or immunotherapeutic drugs or vaccines.

As an increasing number of immune-modifying drugs, monoclonal antibodies, and vaccines are being introduced in the clinic for trial and eventual utilization, there is an increasing risk for the induction of off-target inflammation. Advantageously, the in vitro method o enables a real-time assessment of such responses targeting the heart during the clinical testing phase of novel reagents in development, as well as during approved clinical practice for those reagents with an increased risk profile and/or for patients whose characteristics present increased risk for off-target toxicity.

The in vitro method is suitable to be applied for heart transplantation monitoring, as the detected cardiac antigen-specific IgG antibodies may represent a real-time proxy of rejection of the transplanted heart.

The means suitable for performing the in vitro method are assembled into a kit in order to facilitate the use thereof.

A further non-claimed aspect is a kit for the diagnosis and/or for predicting the risk for developing heart failure (HF) in a human being, in a dog or in a cat.

The one or more isolated antigenic peptides in the kit consist of an amino acid sequence selected from the group consisting of SEQ ID NOs. 1, 2, 3 and 4 of a human 14-3-3 protein; SEQ ID NO. 5 of human small nuclear ribonucleoprotein Sm D1 (SNRPD1); SEQ ID NO. 6 of human ATP synthase subunit O, mitochondrial precursor (ATP5O); SEQ ID NO. 7 of human toll-like receptor 5 precursor (TLR5), and any combination thereof.

The one or more isolated antigenic peptides in the kit consist of an amino acid sequence selected from the group consisting of SEQ ID NOs. 1, 2, 3 and 4 of canine 14-3-3 protein; SEQ ID NO. 8 of canine monocarboxylate transporter 3 and 4 proteins (MCT3 and MCT4); SEQ ID NO. 5 of canine SNRPD1; SEQ ID NO. 9 of canine ATP5O; SEQ ID NO. 10 of canine dihydropyrimidinase (DHP) protein, and any combination thereof.

The one or more isolated antigenic peptides in the kit consists of an amino acid sequence selected from the group consisting of SEQ ID NOs. 1, 2, 3 and 4 of feline 14-3-3 protein; SEQ ID NO. 8 of feline MCT3 and MCT4 proteins; SEQ ID NO. 11 of feline vomeronasal 1 receptor felCatV1R6 protein; SEQ ID NO. 12 of feline transmembrane emp24 domain-containing protein 6; SEQ ID NO. 5 of feline SNRPD1; SEQ ID NO. 13 of feline ATP5O; SEQ ID NO. 10 of feline DHP protein, and any combination thereof.

In the kit the one or more antigenic peptides are immobilized on a solid support. Non-limiting examples of suitable solid supports are the wells of a microtitre plate, the surface of a microparticle such as a latex, polystyrene, silica, chelating sepharose or magnetic beads, membranes, strips or chips.

Further, the kits contain detection means as described above in connection with the in vitro diagnostic methods.

Further experimental studies carried out by the present inventors, which are illustrated in detail in the experimental section that follows, revealed that healthy mice fed with the peptide antigens, when subjected to Transverse Aortic Constriction (TAC), experienced less severe cardiac disease in comparison with control animals treated with phosphate-buffered saline only. Surprisingly, such findings show that the peptide antigens are capable of inducing oral tolerance when administered to an animal, which leads to a preventive protection from the severity of heart failure.

Hence, the isolated peptides, alone or in combination with each other, are particularly suitable for use in orally administered treatment and/or prevention of heart disease.

Accordingly, a further non-claimed aspect is the isolated peptide as above defined for use in the therapeutic treatment of heart failure and/or in the prevention of heart failure in a subject in need thereof, said treatment comprising oral administration of the peptide to the subject, thereby inducing oral tolerance to said peptide.

In the context of the present description, the term "oral tolerance" is intended to mean an active process by which the immune system does not respond with an inflammatory reaction to an orally administered antigen. More particularly, the human body tolerates antigens in the gastro-intestinal tract, most likely as a necessary requirement for maintaining gut flora and digesting food.

The oral tolerance process is considered to occur via the induction of induced immunosuppressive Treg (iTreg) cells that may respond to these antigens (Larché, M (2014), "Mechanisms of peptide immunotherapy in allergic airways disease", Ann Am Thorac Soc, 11 Suppl 5 S292-6; Chen, X, et al. (2017), "Oral administration of visceral adipose tissue antigens ameliorates metabolic disorders in mice and elevates visceral adipose tissue-resident CD4(+)CD25(+)Foxp3(+) regulatory T cells", Vaccine).

Without wishing to be bound by any theory, the inventors believe that oral tolerance induction to the peptide antigens driving the adaptive response that mediate heart failure disease may lead to the generation of antigen-specific immunosuppressive Treg and iTreg cells. These cells act by suppressing any adaptive immune response mediated by pro-inflammatory T cells and B cells leading to heart failure disease, at the time when the deleterious response is initiated.

The method can prevent the onset of disease or reduce its severity. In the preventative context, the method can delay or prevent the onset of one or more symptoms of heart failure.

In the treatment context, the method can delay or prevent the progression of one or more symptoms of heart failure.

The subject in need of the treatment with the peptide can be a human patient or a non-human patient. The non-human patient can be a non-human mammal, for example a dog or a cat. Several strains of domestic dogs and cats suffer from congenital cardiac defects leading to premature onset cardiac dysfunction.

A still further non-claimed aspect is a pharmaceutical composition comprising at least one isolated peptide antigen as well as a pharmaceutically acceptable carrier, vehicle or diluent. The selection of the carrier, vehicle or diluent as well as of any other excipient required for the preparation of the desired pharmaceutical dosage form falls within the ability of the person skilled in the art.

The above defined pharmaceutical composition is preferably a tolerizing vaccine suitable to be administered via the oral route. As known in the art, a tolerizing vaccine is capable of inducing in a subject an antigen-specific immune tolerance.

Preferably, the tolerizing vaccine does not comprise any adjuvant, i.e. any substance capable to specifically trigger antigenicity and inflammation.

Oral formulations generally include an inert diluent or an edible carrier. They can be enclosed in gelatin capsules or compressed into tablets. For the purpose of oral therapeutic administration, the active compound can be incorporated with excipients and used in the form of tablets, troches, or capsules.

A still further non-claimed aspect is a food, feed or drink product comprising at least one isolated peptide as above defined. The food, feed or drink product may be classified as medical food, food supplement or other classification, and can be any food or drink whose preparation does not alter the integrity of the peptide composition.

The following experimental section is provided purely by way of illustration.

### EXAMPLE 1: Transfer of T cells are sufficient to transfer heart failure (HF) from cardiopathic to healthy mice

In order to assess whether T cells are not only necessary but also sufficient to induce heart failure (HF), the present inventors followed the experimental approach depicted in Figure 1A. Such approach involved transferring T cells from mice that had undergone transverse aortic constriction (TAC)-operation to induce HF (or sham-operation controls) into healthy recipient mice, and examining whether this transfer was sufficient to transfer the disease. Briefly, T cells isolated from heart-draining lymph nodes (LN) of C57BL6/J 8 week-old male mice 4 weeks after TAC- or sham-surgery were transferred into healthy mouse recipients. Recipient C57BL6/J 8 week-old male mice received 1*10⁶ cells via intravenous injection.

Cells isolated from heart draining lymph nodes were stained via FACS-analysis in order to examine the population of T cells isolated from heart-draining lymph nodes and injected into healthy recipients. As shown in Figure 1B, T cells isolated from lymph nodes included cytotoxic CD8+, CD4+ conventional naive T cells and CD4+ conventional effector T cells, and regulatory T cells
Cardiac function of the recipient mice was assessed by echocardiography performed at baseline (before receiving T cells), and 9 and 12 weeks after cell transfer. Fractional shortening (%FS), measured via echocardiography, is an indicator of the left ventricle functionality and it represents the percentage of shortening of the left ventricular diameter between end-diastole and end-systole. Mice that received cells from TAC-operated mice showed a significant decrease of heart functionality measured as %FS, compared to mice receiving T cells from control (sham-operated) mice, signifying a reduction in heart functionality (Figure 1C). Data were plotted as mean ± SEM; solid line represents mice that received cells from TAC-operated mice, dotted line represents mice that received cells from sham-operated mice, (n=6-7) Two-way ANOVA, Bonferroni post-test. * p <0.05.

### EXAMPLE 2: Antigen Discovery Strategy

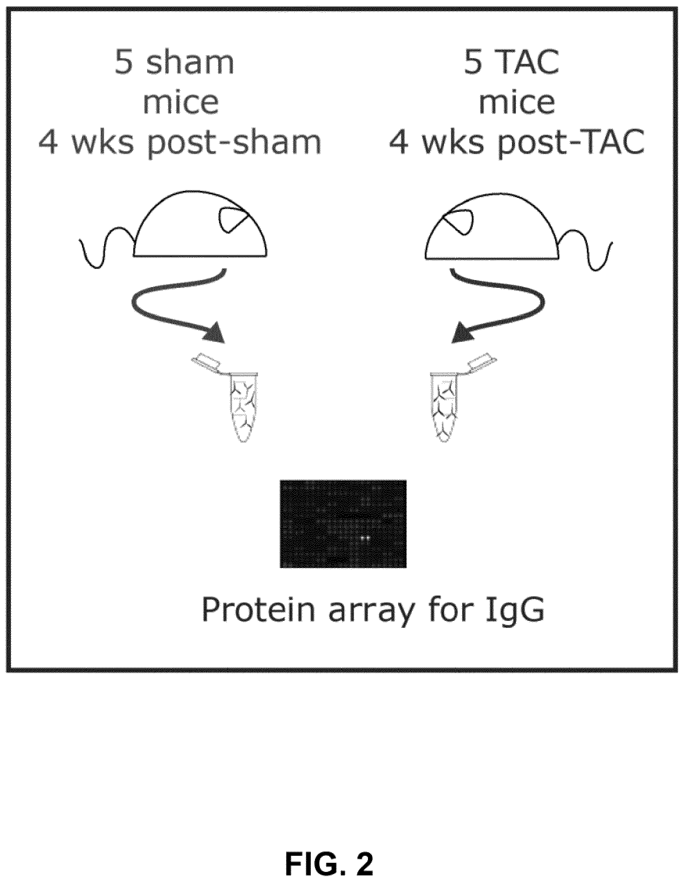

Serum samples were collected from 5 sham-operated mice or 5 TAC-operated mice 4 weeks after sham or TAC surgery. The sera were pooled into two samples, respectively (1 TAC and 1 sham). The pooled serum samples were assayed on a commercial mouse autoantigen and random antigen-discovery array (Figure 2). The bound antibodies were then detected by anti-IgG reagents. The IgG antibody signal was quantified for each hit and the sequence of each recognized peptide (i.e. the sequence of the random oligopeptide spotted on the array) was reported for further analysis, along with the corresponding signal strength. By detecting only IgG antibodies, the inventors narrowed the analysis to the IgG-switched antibodies, which had received T cell help. Importantly, the mice used in the experiment were all congenic, i.e. genetically identical.

### EXAMPLE 3: Pipeline for antigen discovery. Bioinformatic analysis strategy for the differential analysis of the array readout

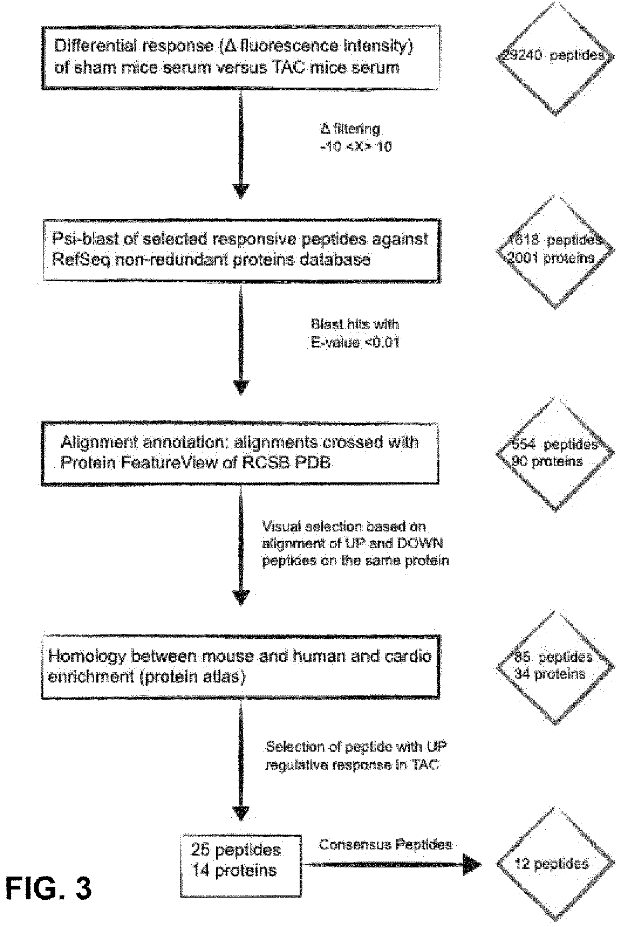

With the aim of identifying peptide antigens driving T cells involved in HF pathology, the present inventors set up the discovery strategy as illustrated in Figure 3.

To perform a differential analysis of the array readout, the inventors ordered each random oligopeptide on the basis of differential ability to produce a hit (TAC IgG binding signal - positive ("up") signals) when tested with TAC pooled serum and no hit when tested with sham pooled serum (sham IgG binding signal - negative "down" signals).

In detail, epitopes recognized by antibodies were selected with a user-defined threshold of x>10 (x = the average and corrected fluorescence intensity changes between Sham mouse serum and TAC mouse serum). The identified oligopeptides (i.e. epitopes) were used to predict potential antigenic proteins using the PSI-BLAST (Position-Specific Iterative Basic Local Alignment Search Tool) from the National Center for Biotechnology Information, which is a protein sequence similarity search program, and the RefSeq non-redundant proteins database (organism Mus musculus (taxid: 10090)). PSI-BLAST parameters were set as default. Candidate proteins corresponding to each identified epitope were selected in according to an E-value < 0.01.

In order to guarantee that identified proteins were relevant for human disease, the hits were filtered so as to select only those proteins for high homology to human proteins. The list of proteins or protein domains with "up" and no "down" hits was tested for sequence identity (at protein level) to human. The homology with human was evaluated performing PSI-BLAST against the RefSeq non-redundant protein database (organism Human (taxid: 9606)). Proteins with a percentage of alignment higher than 67% were selected. Then, cardiac enrichment of each protein was evaluated using Human Protein Atlas database (https://www.proteinatlas.org/).

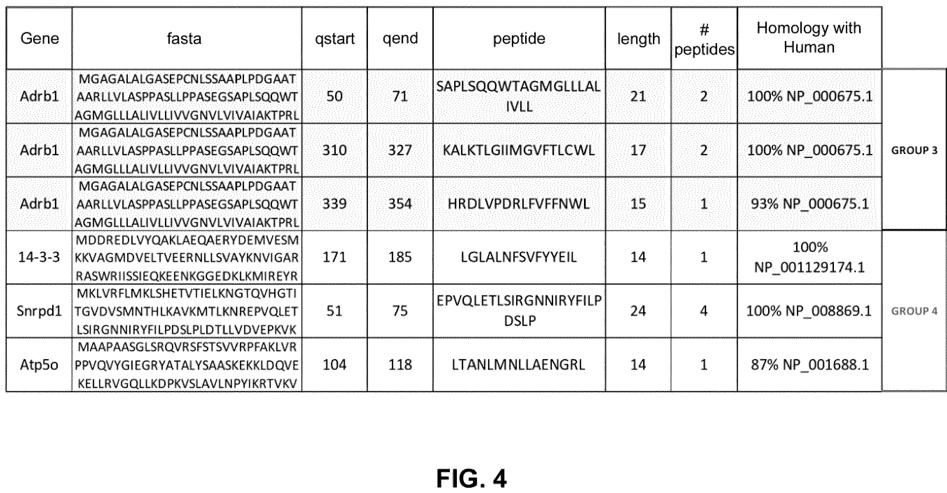

Peptides identified as end-results of the above-described process are listed in the Table of Figure 4. The table contains the following information: name of the mouse gene encoding for the whole protein, amino acid FASTA sequence of the entire protein of origin (sequence not fully visible in the graph), start and end aminoacid number of the identified peptides within each protein of origin, aminoacid sequence of the identified peptide, peptide length, number of source peptides spotted on the array generating each peptide hit, percentage of homology of the peptide with the homologous human protein.

The three peptides composing Group 3 are derived from mouse beta-1 adrenergic receptor protein (SEQ ID NO. 66), and consist of the amino acid sequences designated as SEQ ID NO. 15 (SAPLSQQWTAGMGLLLALIVLL), SEQ ID NO. 16 (KALKTLGIIMGVFTLCWL), and SEQ ID NO. 17 (HRDLVPDRLFVFFNWL), respectively. The beta-1 adrenergic receptor is known to be an autoantigen driving responses in autoimmune myocarditis (Caforio, AL, et al. (2002), "Circulating cardiac autoantibodies in dilated cardiomyopathy and myocarditis: pathogenetic and clinical significance", Eur J Heart Fail, 4 411-17; Basavalingappa, RH, et al. (2017), "β1-Adrenergic Receptor Contains Multiple IAk and IEk Binding Epitopes That Induce T Cell Responses with Varying Degrees of Autoimmune Myocarditis in A/J Mice", Front Immunol, 8 1567). As it is known that peptides from this protein can lead to autoimmune response targeting the heart, group 3 functioned as an internal positive control in the discovery method, validating the functionality of the process.

As shown in Figure 4, Group 4 includes a peptide consisting of amino acid sequence SEQ ID NO. 1, which derives from mouse 14-3-3 protein (SEQ ID NO. 70), a peptide consisting of amino acid sequence SEQ ID NO. 5, which derives from mouse Snrpd1 protein (SEQ ID NO. 79), and a peptide consisting of amino acid sequence SEQ ID NO. 14, which derives from mouse Atp5O protein (SEQ ID NO. 80).

### EXAMPLE 4: Immunization protocol for assessing the cardio-specific immunogenicity of the peptides

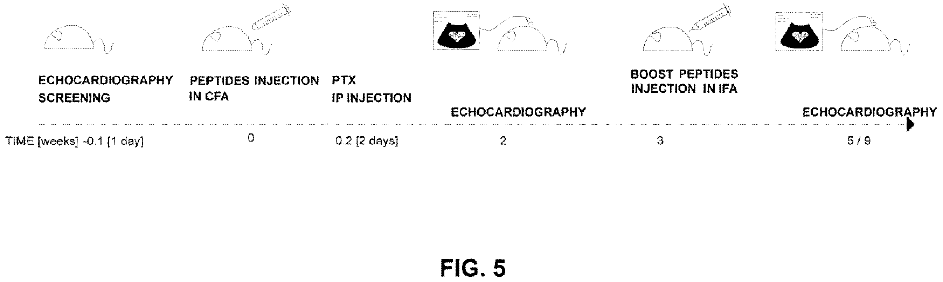

In order to validate the functional relevance of identified antigens recognized by T cells driving HF, the inventors conducted the experiments as described below and schematically represented in Figure 5.

Briefly, C57BL6/J 8-week-old male mice were screened, via echocardiography, at baseline for heart functionality and randomly divided into three experimental groups. Mice belonging to the negative control group (CTR-, only CFA) were injected subcutaneously (immunized) with 100µl Complete Freud's Adjuvant (CFA) at day 0 and boosted with 100µl of Incomplete Freud's Adjuvant (IFA) on day 21, without any antigen. Mice belonging to group 3 (CTR+) were injected subcutaneously (immunized) with 100µg of each of the three peptides derived from beta-adrenergic receptor (SEQ ID NOs. 15-17) emulsified in Complete Freud's Adjuvant (CFA) at day 0 and boosted with 100µg of the three peptides emulsified in Incomplete Freud's Adjuvant (IFA) on day 21. Mice belonging to group 4 (Group 4) were injected subcutaneously (immunized) with 100µg of each of the three peptides of SEQ ID NOs. 1, 5 and 14, derived from murine 14-3-3, Snrpd1 and, Atp5o proteins, emulsified in Complete Freud's Adjuvant (CFA) at day 0 and boosted with 100µg of each of the three peptides emulsified in Incomplete Freud's Adjuvant (IFA) on day 21. On day 0 and day 2, all the mice were intraperitoneally injected with 200ng of Pertussis Toxin (PTX), as per standard immunization protocols. Heart functionality of all mice was analyzed via echocardiography at 2, 5 and 9 weeks after the first subcutaneous injection.

The immunization experiments of healthy mice with peptides derived from beta-adrenergic receptor and from 14-3-3, Snrpd1, and Atp5o lead to cardiac dysfunction. As shown in the bar graphs of Figures 6A and 6B, mice immunized with peptides derived from beta-adrenergic receptor (CTR+) and from 14-3-3, Snrpd1, and Atp5o proteins (Group 4) displayed a significant reduction of cardiac functionality, measured via echocardiography analysis at 2, 5 and 9 weeks after the first subcutaneous injection. Left ventricle functionality was measured as %FS (Figure 6A) and Ejection Fraction (%EF) (Figure 6B), which measure the volumetric capacity of the left ventricle to pump blood. Data were plotted as mean ± SEM, (n= 4-6). Two-way ANOVA and Tukey's multiple comparison test. * p <0.05, ** p <0.01.

### EXAMPLE 5: Immunization of healthy mice with single peptides derived from 14-3-3, Snrpd1, and Atp5o induces cardiac dysfunction.

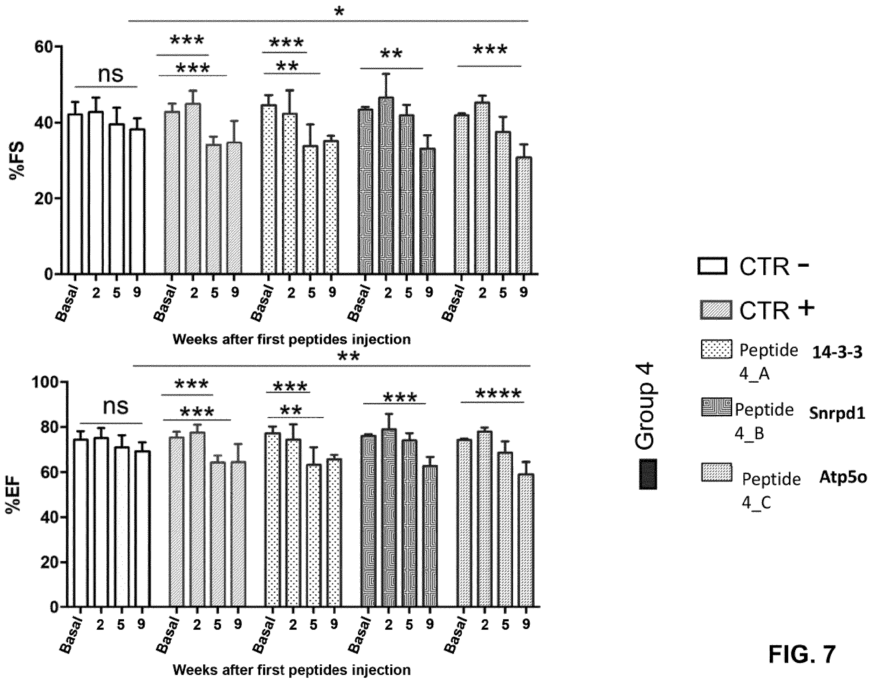

C57BL6/J 8-week-old male mice were screened at baseline for heart functionality, via echocardiography, and randomly divided into 5 experimental groups. Mice belonging to control group (CTR-, only CFA) were injected subcutaneously (immunized, but without antigen: negative control) with 100µl Complete Freud's Adjuvant (CFA) at day 0 and boosted with 100µl of Incomplete Freud's Adjuvant (IFA) on day 21. Mice belonging to group 3 (CTR+) were injected subcutaneously (immunized, known antigen: positive control) with 100µg of each of the three peptides (SEQ ID NOs. 15-17) derived from beta-adrenergic receptor and listed in the Table of Figure 4, emulsified in Complete Freud's Adjuvant (CFA) at day 0 and boosted with 100µg of the three peptides emulsified in Incomplete Freud's Adjuvant (IFA) on day 21. Mice belonging to group 4_A (Peptide 4_A) were injected subcutaneously (immunized) with 100µg of the peptide of SEQ ID NO. 1, derived from 14-3-3 protein, emulsified in Complete Freud's Adjuvant (CFA) at day 0 and boosted with 100µg of each of the three peptides emulsified in Incomplete Freud's Adjuvant (IFA) on day 21. Mice belonging to group 4_B (Peptide 4_B) were injected subcutaneously (immunized) with 100µg of the peptide of SEQ ID NO. 5, derived from Snrpd1 protein, emulsified in Complete Freud's Adjuvant (CFA) at day 0 and boosted with 100µg of each of the three peptides emulsified in Incomplete Freud's Adjuvant (IFA) on day 21. Mice belonging to group 4_C (Peptide 4_C) were injected subcutaneously (immunized) with 100µg of the peptide of SEQ ID NO. 14, derived from Atp5o protein, emulsified in Complete Freud's Adjuvant (CFA) at day 0 and boosted with 100µg of each of the three peptides emulsified in Incomplete Freud's Adjuvant (IFA) on day 21. On day 0 and day 2, all the mice were intraperitoneally injected with 200ng of Pertussis Toxin. Heart functionality of all mice was analyzed via echocardiography at 2, 5 and 9 weeks after the first subcutaneous injection. In Figure 7, the results of echocardiography analysis of cardiac functionality parameters %FS and %EF of C57BL6/J mice immunized with the peptide of SEQ ID NO. 1 (14-3-3 protein), the peptide of SEQ ID NO. 5 (Snrpd1 protein), or the peptide of SEQ ID NO. 14 (Atp5o protein) are shown. Mice immunized with peptides derived from beta-adrenergic receptor and from 14-3-3, Snrpd1, and Atp5o proteins showed a significantly reduced %FS and %EF. Data are plotted as mean ± SEM, (n= 4-6). Two-way ANOVA and Tukey's multiple comparison test with matching subjects. * p <0.05, ** p <0.01, ***p<0.001.

### EXAMPLE 6: Validation of functional relevance of identified antigens recognized by T cells driving HF

The present inventors conducted dedicated experiments in order to assess whether human Heart Failure patients develop IgG responses against the newly-identified antigens, and thus whether the T cell-dependent antibody response to the antigens can be used to differentially identify HF patients.

ELISA assays were performed using as capture molecules the peptides of SEQ ID NO. 1 (peptide 4_A), SEQ ID NO. 5 (peptide 4_B), and SEQ ID NO. 14 (peptide 4_C), either alone or in combination. A total of 100 ng of either the peptide 4_A, peptide 4_B, or peptide 4_C, or of these peptides in combination was coated on 96-well plates overnight and blocking of non-specific binding was performed. Healthy or heart failure patient human serum samples were then applied and incubated for 1 hour. Anti-human IgG-HRP was used to detect the bound IgG antibodies present in the serum. Addition of TMB (3,3',5,5'-tetramethylbenzidine) to the wells created a colorimetric reaction, which is proportional to the presence of bound antibodies. The intensity of the colorimetric reaction was measured and the absorbance of each sample, after blank subtraction, was plotted (Figure 8). Data were plotted as mean ± SEM, (n=2); unpaired t-test. * p <0.05.

The results of the ELISA assays described above show that the peptides , either alone or in combination, enabled the detection of a significant difference between HF patients and healthy controls.

### EXAMPLE 7: Application of identified antigens recognized by T cells driving HF in preventive therapy experiment

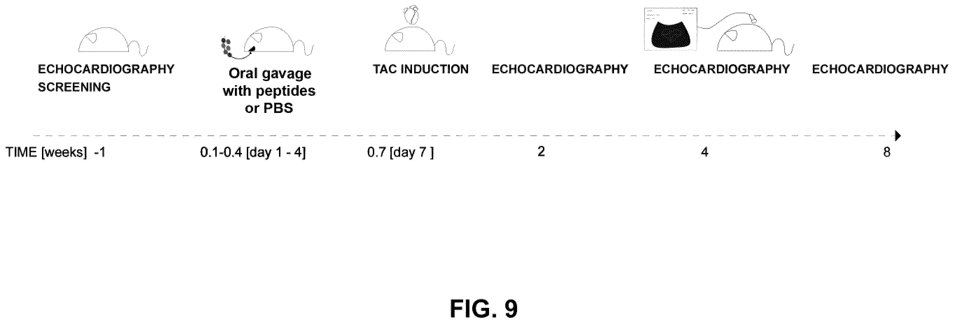

To assess preventive protection potential of oral tolerance with the newly identified peptides from the development of Heart Failure, the inventors pursued an oral tolerance protocol to induce cardio-specific tolerance for the peptides, followed by active induction of Heart Failure via TAC. A schematic representation of the protocol pursued by the inventors is shown in Figure 9.

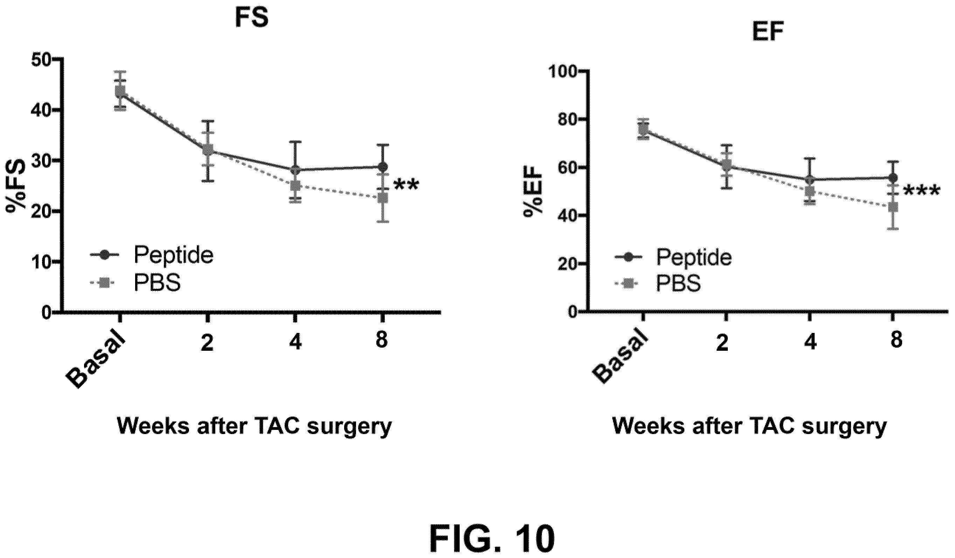

Briefly, C57BL6/J 8-week-old male mice were screened at baseline via echocardiography. Each mouse was orally fed with 0.8mg of each peptide from group 4: the peptide of SEQ ID NO. 1 (14-3-3 protein), the peptide of SEQ ID NO. 5 (Snrpd1 protein), and the peptide of SEQ ID NO. 14 (Atp5o protein) (i.e. 0.8mg per day of each peptide, thus 2.4 mg/day in total), diluted in 600µl of Phosphate Buffered-Saline (PBS) or plain PBS (as control) via oral feeding (oral gavage) for 4 days in a row. Three days after the end of this procedure, on day 7, the mice underwent TAC surgery, to experimentally induce Heart Failure. Echocardiographies were performed on both groups of mice to monitor their cardiac functionality at 2, 4 and 8 weeks after TAC-surgery. As shown in Figure 10, oral administration of peptides prevented cardiac dysfunction in TAC-operated mice. Mice that orally received the identified peptides (peptides of SEQ ID NO. 1, 5 and 14) with the tolerization protocol depicted above, showed significantly better cardiac functionality (measured as %FS and %EF, solid line) 8 weeks after surgery compared to mice treated with PBS alone (dotted line). Data were plotted as mean ± SEM, (n= 8-11). Two-way ANOVA and Tukey's multiple comparison test with matching subjects. ** p <0.01, ***p<0.001.

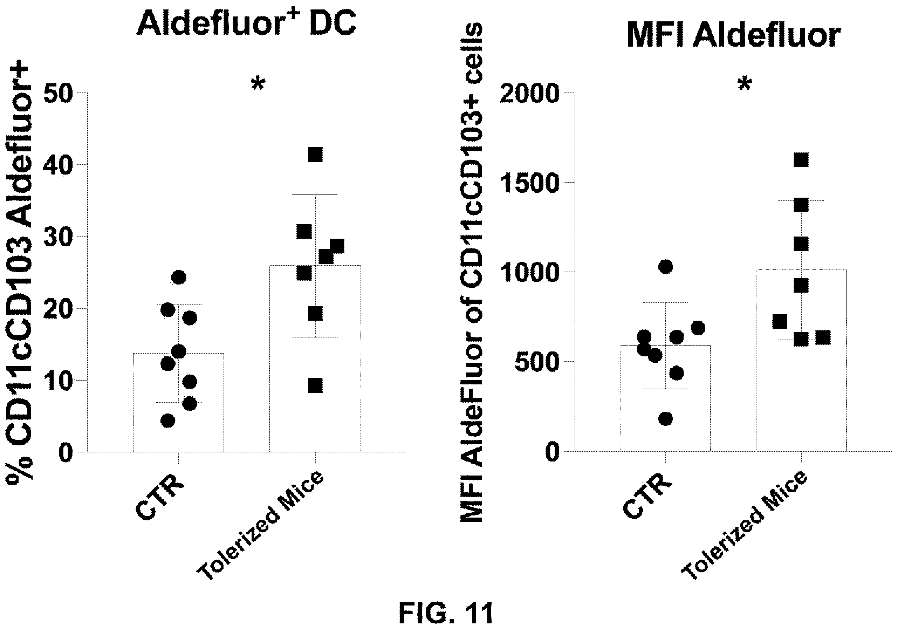

Moreover, the present inventors found that oral administration of peptides induces oral tolerance towards the fed peptides via retinoic acid production, known to promote the generation of induced immunosuppressive regulatory T cells. Briefly, eight weeks after oral tolerance induction in TAC-operated mice, mesenteric lymph nodes (MLN) were collected and dendritic cells (DC) were isolated for FACS analysis. Aldehyde dehydrogenase (ALDH) production of retinoic acid from the DC was measured via Aldefluor Fluorescent dye. FACS analysis of DC isolated from MLN of mice fed with the peptides of SEQ ID NO. 1, 5 and 14 showed a significant increase in ALDH activity (Figure 11), which is a proxy for retinoic acid production. Retinoic acid produced by tolerogenic DC contributes to Regulatory T cell (Treg) generation, essential for tolerance induction. The bar graphs in Figure 11 illustrate that both percentage of ALDH positive DC and the mean fluorescence intensity (MFI), induced by the presence of the fluorescent dye, in mice orally fed with the peptides compared to mice fed with PBS alone showed a significant increase. Data are plotted as mean ± SEM, (n= 7-8). Unpaired t-test. * p <0.05.

## Claims

1. A method for identifying a peptide antigen which is relevant to a human or veterinary non-autoimmune disease involving T cell activation, said method comprising the following steps:
(i) contacting a first biological fluid sample containing IgG immunoglobulins from a first non-human animal affected by the non-autoimmune disease with a microarray comprising a plurality of isolated or synthesized antigenic peptides, each peptide having a pre-determined location in the microarray and comprising an amino acid sequence of a protein from the non-human animal;
(ii) detecting the binding of one or more of the IgG immunoglobulins present in the first sample with one or more of the antigenic peptides in the microarray to provide a first IgG-bound peptide profile;
(iii) comparing the first IgG-bound peptide profile to a second IgG-bound peptide profile generated by contacting a microarray as defined in step (i) with a second biological fluid sample containing IgG immunoglobulins from a second non-human animal, wherein the second non-human animal is not affected by the non-autoimmune disease and wherein the first and second non-human animals belong to the same species and are congenic animals;
(iv) identifying the one or more IgG-bound peptides which are present in the first IgG-binding profile and are not present in the second IgG binding profile;
(v) querying a database of animal protein sequences using the amino acid sequence of each IgG-bound peptide identified in step (iv) as the query in order to select one or more animal proteins comprising a peptide sequence which has an amino acid sequence identity to the amino acid sequence of each of the queried peptides comprised between 67% and 100%, wherein the animal protein sequences are from an animal belonging to a species which is different from the first and second non-human animals of step (iii);
(vi) identifying as peptide antigens relevant to the non-autoimmune disease the peptide sequences which have from 67% to 100% amino acid sequence identity selected in step (v) and which are comprised of an animal protein expressed in a tissue affected by the non-autoimmune disease
wherein the first and second non-human animals are mice and the first non-human animal is a mouse model of the disease,
wherein the non-autoimmune disease is selected from the group consisting of heart failure (HF), cardiac diseases, vascular diseases, atherosclerosis, vascular stenosis, non-type 1 diabetes, metabolic syndrome, obesity, Amyotrophic Lateral Sclerosis-ALS, Alzheimer's Disease, Dementia, Age-related Dementia, Mild Cognitive Decline, Parkinson's Disease, and any combination thereof.

2. The method according to claim 1, wherein the heart failure is hemodynamically induced heart failure.

3. The method according to claim 1 or 2, wherein the combination of non-autoimmune diseases is a multimorbidity condition.

4. The method according to any of claims 1 to 3, wherein the animal in step (v) is a human being, a dog or a cat.

## Patentansprüche

1. Verfahren zur Identifizierung eines Peptidantigens, das für eine menschliche oder tierische Nicht-Autoimmunerkrankung relevant ist, die eine T-Zellen-Aktivierung beinhaltet, wobei das Verfahren die folgenden Schritte umfasst:
(i) Inkontaktbringen einer ersten biologischen Fluidprobe, die IgG-Immunglobuline von einem ersten nicht-menschlichen Tier enthält, das von der Nicht-Autoimmunerkrankung betroffen ist, mit einem Mikroarray, das eine Vielzahl von isolierten oder synthetisierten antigenen Peptiden umfasst, wobei jedes Peptid eine vorbestimmte Position im Mikroarray aufweist und eine Aminosäuresequenz eines Proteins des nicht-menschlichen Tieres umfasst;
(ii) Nachweisen der Bindung von einem oder mehreren der in der ersten Probe vorhandenen IgG-Immunglobuline mit einem oder mehreren der antigenen Peptide im Mikroarray, um ein erstes IgG-gebundenes Peptidprofil zu erstellen;
(iii) Vergleichen des ersten IgG-gebundenen Peptidprofils mit einem zweiten IgG-gebundenen Peptidprofil, das durch Inkontaktbringen eines Mikroarrays, wie in Schritt (i) definiert, mit einer zweiten biologischen Fluidprobe, die IgG-Immunglobuline von einem zweiten nicht-menschlichen Tier enthält, erzeugt wird, wobei das zweite nicht-menschliche Tier nicht von der Nicht-Autoimmunerkrankung betroffen ist und wobei das erste und das zweite nicht-menschliche Tier zu derselben Spezies gehören und kongene Tiere sind;
(iv) Identifizieren des einen oder der mehreren IgG-gebundenen Peptide, die in dem ersten IgG-Bindungsprofil vorhanden sind und in dem zweiten IgG-Bindungsprofil nicht vorhanden sind,
(v) Abfragen einer Datenbank mit tierischen Proteinsequenzen unter Verwendung der Aminosäuresequenz jedes in Schritt (iv) identifizierten IgG-gebundenen Peptids als Abfrage, um ein oder mehrere tierische Proteine auszuwählen, die eine Peptidsequenz umfassen, die eine Aminosäuresequenzidentität mit der Aminosäuresequenz jedes der abgefragten Peptide zwischen 67 % und 100 % aufweist, wobei die tierischen Proteinsequenzen von einem Tier stammen, das zu einer Spezies gehört, die sich von den ersten und zweiten nicht-menschlichen Tieren von Schritt (iii) unterscheidet;
(vi) Identifizieren als Peptidantigene, die für die Nicht-Autoimmunerkrankung relevant sind, der Peptidsequenzen, die eine Aminosäuresequenzidentität von 67 % bis 100 % aufweisen, die in Schritt (v) ausgewählt wurden, und die aus einem tierischen Protein bestehen, das in einem von der Nicht-Autoimmunerkrankung betroffenen Gewebe exprimiert wird, wobei die ersten und zweiten nicht-menschlichen Tiere Mäuse sind und das erste nicht-menschliche Tier ein Mausmodell der Erkrankung ist,
wobei die Nicht-Autoimmunerkrankung ausgewählt ist aus der Gruppe bestehend aus Herzinsuffizienz (HF), Herzerkrankungen, Gefäßerkrankungen, Atherosklerose, Gefäßverengung, Nicht-Grad-1-Adipositas, Amyotrophe Lateralsklerose (ALS), Alzheimer-Krankheit, Demenz, altersbedingte Demenz, leichter kognitiver Verfall, Parkinson-Krankheit und jede Kombination davon.

2. Verfahren nach Anspruch 1, wobei die Herzinsuffizienz eine hämodynamisch bedingte Herzinsuffizienz ist.

3. Verfahren nach Anspruch 1 oder 2, wobei die Kombination von Nicht-Autoimmunerkrankungen ein multimorbider Zustand ist.

4. Verfahren nach einem der Ansprüche 1 bis 3, wobei das Tier in Schritt (v) ein Mensch, ein Hund oder eine Katze ist.

## Revendications

1. Procédé d'identification d'un antigène peptidique qui est pertinent pour une maladie non-autoimmune humaine ou vétérinaire impliquant l'activation de lymphocytes T, ledit procédé comprenant les étapes suivantes consistant à :
(i) mettre en contact un premier échantillon de fluide biologique contenant des immunoglobulines IgG d'un premier animal non humain affecté par la maladie non- autoimmune avec un microréseau comprenant une pluralité de peptides antigéniques isolés ou synthétisés, chaque peptide ayant une place pré-déterminée dans le microréseau et comprenant une séquence d'acides aminés d'une protéine de l'animal non humain ;
(ii) détecter la liaison d'une ou de plusieurs des immunoglobulines IgG présentes dans le premier échantillon avec un ou plusieurs des peptides antigéniques dans le microréseau pour fournir un premier profil de peptides liés à IgG ;
(iii) comparer le premier profil de peptides liés à IgG avec un second profil de peptides liés à IgG produit en mettant en contact un microréseau tel que défini dans l'étape (i) avec un second échantillon de fluide biologique contenant des immunoglobulines IgG d'un second animal non humain, le second animal non humain n'étant pas affecté par la maladie non-autoimmune et le premier et le second animaux non humains étant de la même espèce et étant des animaux congéniques ;
(iv) identifier les un ou plusieurs peptides liés à IgG qui sont présents dans le premier profil de peptides liés à IgG et ne sont pas présents dans le second profil de peptides liés à IgG,
(v) chercher dans une base de données de séquences protéiques animales en utilisant la séquence d'acides aminés de chaque peptide lié à IgG identifié dans l'étape (iv) comme la recherche pour sélectionner une ou plusieurs protéines animales comprenant une séquence peptidique qui a une identité de séquence d'acides aminés à la séquence d'acides aminés de chacun des peptides recherchés comprise entre 67 % et 100 %, les séquences protéiques animales étant d'un animal appartenant à une espèce qui est différente des premier et second animaux non humains de l'étape (iii) ;
(vi) identifier comme antigènes peptidiques pertinents pour la maladie non-autoimmune les séquences peptidiques qui ont de 67 % à 100 % d'identité de séquence d'acides aminés sélectionnées dans l'étape (v) et qui sont comprises d'une protéine animale exprimée dans un tissu affecté par la maladie non-autoimmune les premier et second animaux non humains étant des souris et le premier animal non humain étant un modèle de souris de la maladie,
la maladie non autoimmune étant choisie dans le groupe constitué par la défaillance cardiaque (HF), les maladies cardiaques, les maladies vasculaires, l'athérosclérose, la sténose vasculaire, le diabète de non type 1, le syndrome métabolique, l'obésité, la sclérose latérale amyotrophique-ALS, la maladie d'Alzheimer, la démence, la démence liée à l'âge, le déclin cognitif léger, la maladie de Parkinson et l'une quelconque de leurs combinaisons.

2. Procédé selon la revendication 1, dans lequel la défaillance cardiaque est une défaillance cardiaque induite hémodynamiquement.

3. Procédé selon la revendication 1 ou 2, dans lequel la combinaison de maladies non-autoimmunes est un état de multimorbidité.

4. Procédé selon l'une quelconque des revendications 1 à 3, dans lequel l'animal dans l'étape (v) est un être humain, un chien ou un chat.
